Europäisches Patentamt

European Patent Office

Office européen des brevets

(11) Numéro de publication: **0 345 892**
**A1**

(12) ## DEMANDE DE BREVET EUROPEEN

(21) Numéro de dépôt: **89201431.7**

(22) Date de dépôt: **05.06.89**

(51) Int. Cl.⁴: **A61B 6/04 , A61B 6/00**

(30) Priorité: **10.06.88 FR 8807776**

(43) Date de publication de la demande:
**13.12.89 Bulletin 89/50**

(84) Etats contractants désignés:
**DE FR GB IT NL**

(71) Demandeur: **PHILIPS SYSTEMES MEDICAUX**
**177, rue de Bezons**
**F-78420 Carrières sur Seine(FR)**
(84) **FR**

Demandeur: **N.V. Philips'**
**Gloeilampenfabrieken**
**Groenewoudseweg 1**
**NL-5621 BA Eindhoven(NL)**
(84) **DE GB IT NL**

(72) Inventeur: **Louiday, André**
**Société CIVILE S.P.I.D. 209 rue de**
**l'Université**
**F-75007 Paris(FR)**

(74) Mandataire: **Hoarau de La Source, Jean Marie**
**Pierre et al**
**S.P.I.D. 209, rue de l'Université**
**F-75007 Paris(FR)**

(54) Dispositif d'examen radiologique.

(57) Dispositif d'examen radiologique comprenant un socle (1) portant une table porte-patient (2), un bloc moteur (10) muni d'un arbre (14) d'entraînement dont l'axe longitudinal est placé transversalement à la table porte-patient, des moyens de déplacement liés au bloc moteur, le moyen de déplacement étant composé du bloc moteur déplaçable sur un support linéaire (11) de la table porte patient, d'une chaîne d'entraînement liée rigidement au bloc moteur (10) et refermée sur lui, la chaîne, lors du déplacement du bloc moteur sur son support linéaire, entraînant une colonne portant un système d'imagerie X, soit en translation soit en rotation.

FIG. 2

EP 0 345 892 A1

## Dispositif d'examen radiologique

L'invention concerne un dispositif d'examen radiologique comportant un socle portant une table porte-patient parallélépipédique, un bloc moteur muni d'un arbre d'entraînement horizontal dont l'axe longitudinal est placé transversalement à la table porte-patient et des moyens de déplacement liés au bloc moteur.

Un tel dispositif d'examen radiologique est connu de la demande de brevet français n° 2 591 466. Dans la demande de brevet citée, il est décrit un dispositif d'examen radiologique comprenant une table porte patient déplaçable longitudinalement, un socle muni de moyens supports et de moyens de déplacements, un moteur fixé au socle et coopérant avec les moyens de déplacement pour déplacer la table par rapport au socle. Le déplacement s'effectue sous l'action du moteur, solidaire du socle et dont le mouvement de rotation est transformé en un mouvement linéaire, transmis à la table, par un système de rouleaux, poulies et courroies. L'arbre du moteur est mis en rotation et entraîne, par l'intermédiaire de pignons crantés, des courroies dont les extrémités sont fixées à la table à déplacer. Les courroies s'enroulent sur différentes poulies et leur déplacement entraîne le déplacement de la table porte-patient. Les différentes poulies et rouleaux sont tenus par des axes supports solidarisés au socle.

Un des inconvénients du dispositif d'examen est la répartition des différentes poulies et rouleaux du moyen de déplacement qui conduit à conférer au socle une dimension importante, parallèlement à l'axe longitudinal de la table. Cette dimension importante du socle constitue une gène, particulièrement dans les dispositifs d'examen où un accès aisé du patient est nécessaire de part et d'autre de la table porte- patient. De plus, la multiplication des poulies et rouleaux conduit à augmenter de manière non négligeable le coût du dispositif d'examen.

Un autre inconvénient réside en ce que ce moyen de déplacement ne peut engendrer qu'un mouvement linéaire.

L'invention a pour but de réaliser un dispositif d'examen radiologique dans lequel un moyen de déplacement peut engendrer alternativement, de façon simple, un mouvement de translation ou un mouvement de rotation pour un déplacement, par rapport à une table, d'une colonne portant un système d'imagerie relativement lourd, système d'imagerie comprenant une source de rayons X et un sériographe.

L'invention se caractérise en ce que le moyen de déplacement est composé du bloc moteur déplaçable sur un support linéaire et longitudinal à la table porte patient, d'une chaîne d'entraînement liée rigidement au bloc moteur et refermée sur lui, la chaîne, lors du déplacement du bloc moteur sur son support linéaire, entraînant une colonne soit en translation sur une glissière longitudinale de la table porte patient, soit en rotation autour d'un axe de colonne, horizontal et transversal à la table, porté par la partie mobile de la glissière, la colonne portant un système d'imagerie composé d'une source de rayons X et d'un sériographe.

Le mouvement longitudinal et le mouvement de rotation alternés sont réalisés par le fait que la chaîne est une boucle refermée sur le bloc moteur, la colonne étant alternativement bloquée soit en rotation soit en translation.

Préférentiellement, au bloc moteur est assujettie une masse morte d'équilibrage, le bloc moteur entraînant la masse morte d'équilibrage à l'opposé de la colonne par rapport au plan de symétrie transversal de la table porte patient. La masse morte d'équilibrage permet de compenser en partie, par le déplacement du bloc moteur, la masse de la colonne et le système d'imagerie. Le bloc moteur se déplace sur un support linéaire de la table porte-patient en opposition du mouvement de translation de la colonne portant le système d'imagerie. Cela permet d'équilibrer de part et d'autre d'un axe central support de la table porte-patient, la masse de la colonne munie du système d'imagerie et la masse du bloc moteur muni de la masse morte d'équilibrage. La masse du bloc moteur est utilisée pour une partie de la masse d'équilibrage globale.

Dans une forme préférentielle de l'invention, la rotation de la colonne est assurée par une portion de couronne sur laquelle s'enroule la chaîne d'entraînement, couronne assujettie à un chariot et liée à la colonne par un pion d'entraînement, le pion d'entraînement étant placé entre l'axe de colonne et la source de rayons X du système d'imagerie. Le pion d'entraînement, placé entre l'axe de colonne et la source de rayons X du système d'imagerie, de masse importante, permet un déplacement aisé en rotation de la colonne. Le moteur exerce un couple relativement réduit, et peut être de puissance nominale réduite.

Dans une forme de l'invention les déplacements de rotation et de translation de la colonne sont assurés par un frein double alterné bloquant soit la rotation de la colonne pour son déplacement en translation, soit la translation de la colonne pour son déplacement en rotation.

Chacun des deux mouvements est engendré par un moteur associé à un frein bloquant alternativement soit la rotation de la colonne autour de l'axe de colonne soit la translation du chariot par rapport à la table porte patient.

La description qui suit, en se référant aux dessins annexés, le tout donné à titre d'exemple non limitatif fera bien comprendre comment l'invention peut être réalisée.

La figure 1 représente le dispositif d'examen radiologique selon l'invention.

La figure 2 donne un schéma explicatif du moyen de déplacement de la colonne portant un système d'imagerie.

La figure 3a est un schéma explicatif du déplacement longitudinal de la colonne.

La figure 3b est un schéma explicatif du déplacement en rotation de la colonne.

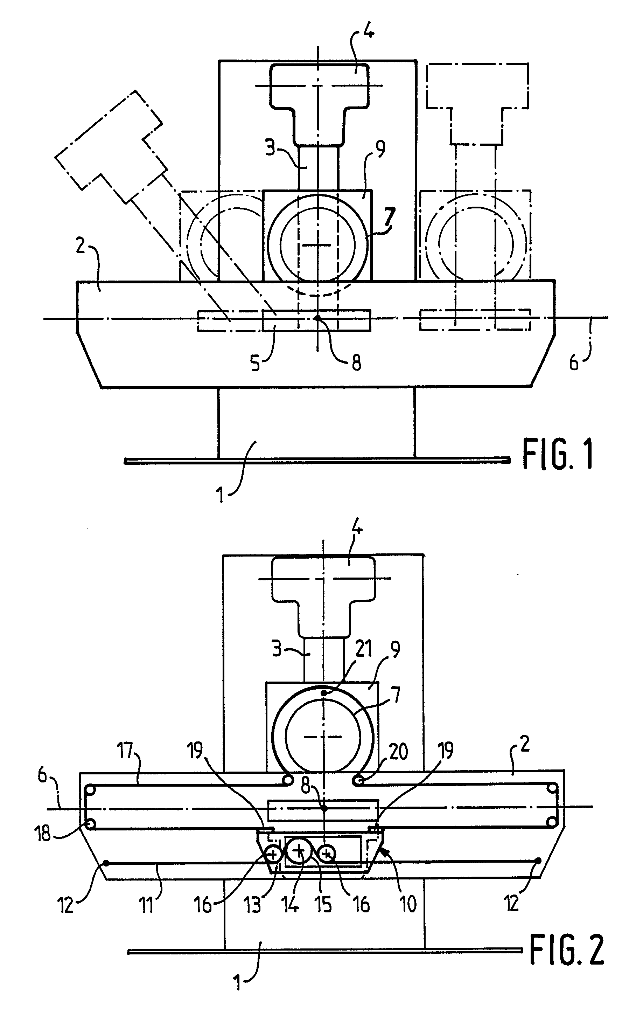

La figure 1 représente un dispositif d'examen radiologique comportant un socle 1 portant une table porte patient 2 basculante et une colonne 3 mobile portant elle-même à une de ses extrémités, au-dessus de la table, une source 4 de rayons X et à l'autre de ses extrémités, aménagé dans la table porte patient 2, un sériographe 5.

La table porte patient 2 basculante autour d'un axe central support, porte une glissière longitudinale, non représentée sur le dessin, dans laquelle coulisse, suivant un axe longitudinal 6 un coulisseau portant un axe de colonne 8 horizontal et transversal à la table porte patient 2, permettant une rotation et un basculement de la colonne 3.

Le déplacement longitudinal du coulisseau auquel est assujetti le sériographe 5 permet un déplacement longitudinal de l'axe de colonne et de ce fait un déplacement du sériographe 5 longitudinalement à la table porte patient 2.

Un chariot 9 se déplaçant longitudinalement à la table porte patient 2 supporte une couronne 7 d'entraînement à rotation de la colonne 3.

La figure 2 est un schéma du moyen de déplacement de la colonne 3, le moyen de déplacement de la colonne est composé d'un bloc moteur 10 déplaçable sur un support linéaire 11 fixé sur la table porte patient 2 par deux de ses extrémités 12, parallèlement à l'axe longitudinal 6 de la table porte patient 2. Le bloc moteur 10 est formé d'un caisson 13 portant un moteur électrique avec son réducteur et dont l'axe d'entraînement 14 est placé horizontalement et perpendiculairement à l'axe longitudinal 6 de la table porte patient 2. L'axe d'entraînement 14 est muni d'une roue dentée d'entraînement 15 assujetti au support linéaire 11 par l'intermédiaire de roues dentées de guidage 16 montées sur le caisson 13 pour permettre le déplacement du bloc moteur le long du support linéaire 11. Le support linéaire 11 est dans l'exemple de réalisation de l'invention, une chaîne support dans laquelle s'engraine la roue dentée d'entraînement 15 et les roues dentées de guidage 16. Le support linéaire 11 du bloc moteur 10 peut être par exemple une crémallière rigide. Le bloc moteur 10 comprend en outre, aménagées dans le caisson 13, des masses mortes d'équilibrage, masses mortes d'équilibrage permettant de compenser les charges de porte à faux provoquées par la colonne 3 et la source 4 de rayons X lorsque celles-ci sont placées de part et d'autre d'un axe central support de la table porte-patient.

De part et d'autre du caisson 13 du bloc moteur 10 sur deux points d'encrage 19 sont fixées les deux extrémités d'une chaîne d'entraînement 17, chaîne d'entraînement 17 se refermant sur le bloc moteur 10 et s'engrenant d'une part sur des roues dentées de renvoi 18 montées en rotation libre aux deux extrémités de la table porte patient, et d'autre part autour de la couronne 7 dentée du chariot 9 par l'intermédiaire de galets 20 montés sur ledit chariot 9.

La colonne 3 est liée à la couronne 7 par un pion 21 d'entraînement. Un frein double alterné, non représenté sur les dessins, assure soit le blocage de la colonne 3 en solidarisant la couronne 7 et le chariot 9, soit le blocage du chariot en le solidarisant à la table porte patient 2.

Le dispositif d'examen radiologique fonctionne de la manière qui suit.

Dans une situation 1, comme représenté sur la figure 2, le bloc moteur 10 et le chariot 9 sont placés dans l'axe transversal de la table et du dispositif d'examen, les charges de l'ensemble table porte patient 2, bloc moteur 10 et colonne 3, sont alors portées directement par l'axe central support de la table porte patient 2.

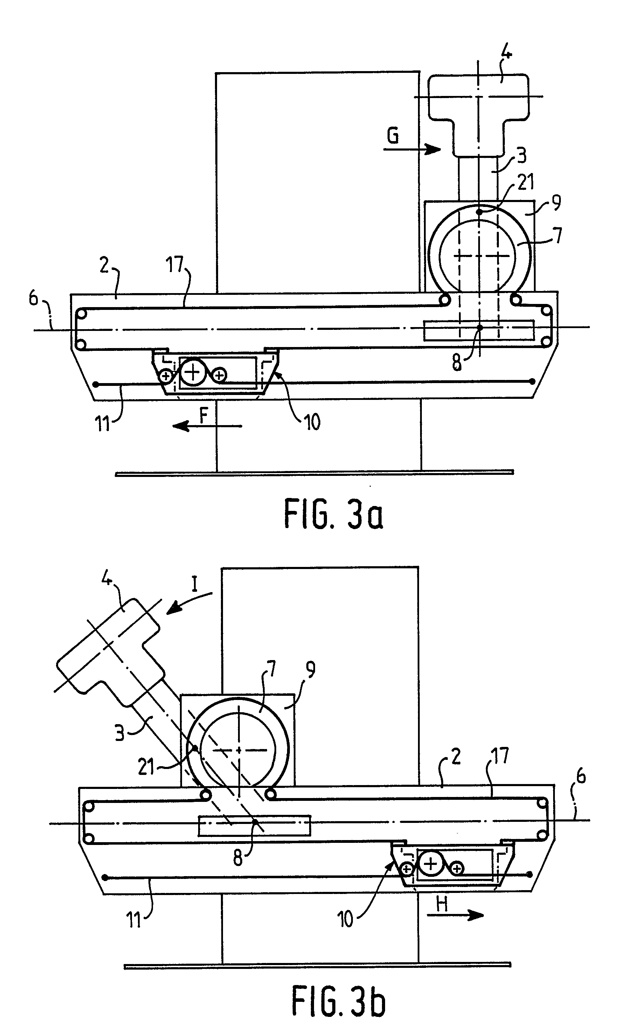

Dans une situation 2, comme représenté sur la figure 3a, le frein double alterné solidarise la couronne 7 et le chariot 9. L'entraînement du bloc moteur 10 suivant la flèche F par rotation du moteur, le long du support linéaire 11, déplace par l'intermédiaire de la chaîne d'entraînement 17, le chariot 9 ainsi que la colonne 3 dans la direction de la flèche G, l'axe longitudinal de la colonne 3 ayant constamment une même direction.

Le bloc moteur 10 se déplace à l'opposé du chariot et de la colonne, par rapport à l'axe central support de la table porte patient 2, ce qui permet au bloc moteur 10 muni de sa masse morte d'équilibrage de compenser le couple engendré par le poids de la colonne par rapport à l'axe central support de la table porte patient 2.

Dans une situation 3, comme représenté sur la figure 3b, le frein double alterné solidarise le chariot 9 à la table porte patient 2 par exemple dans une position indiquée sur la figure. Le déplacement du bloc moteur le long du support linéaire 11, suivant la flèche H, entraîne à rotation la couronne 7 par l'intermédiaire de la chaîne d'entraînement 17. La rotation de la couronne 7 permet l'inclinaison, suivant la flèche I, de la colonne 3 qui lui est assujettie, par l'intermédiaire du pion 21 d'entraînement. La colonne bascule autour de l'axe de colonne 8. Dans cette position le bloc moteur muni de ses masses d'équilibrage compense partiellement le porte à faux de la masse de la colonne et de la source de rayons X.

La couronne 7 et la position du pion d'entraînement 21 permet avantageusement de réduire la puissance nominale du moteur car la force mécanique engendrée par la chaîne d'entraînement et le bloc moteur lors de l'inclinaison de la colonne est réduit par l'importance du bras de levier formé par la distance séparant le pion d'entraînement 21 de l'axe de colonne 8.

**Revendications**

1. Dispositif d'examen radiologique comportant un socle portant une table porte-patient parallélépipédique, un bloc moteur muni d'un arbre d'entraînement horizontal dont l'axe longitudinal est placé transversalement à la table porte-patient, des moyens de déplacement liés au bloc moteur, caractérisé en ce que le moyen de déplacement est composé du bloc moteur déplaçable sur un support linéaire et longitudinal à la table porte patient, d'une chaîne d'entraînement liée rigidement au bloc moteur et refermée sur lui, la chaîne, lors du déplacement du bloc moteur sur son support linéaire, entraînant une colonne soit en translation sur une glissière longitudinale de la table porte patient, soit en rotation autour d'un axe de colonne, horizontal et transversal à la table, porté par la partie mobile de la glissière, la colonne portant un système d'imagerie composé d'une source de rayons X et d'un sériographe.

2. Dispositif d'examen selon la revendication 1, caractérisé en ce qu'au bloc moteur est assujettie une masse morte d'équilibrage, le bloc moteur entraînant la masse morte d'équilibrage à l'opposé de la colonne par rapport au plan de symétrie transversal de la table porte patient.

3. Dispositif d'examen selon les revendications 1 et 2, caractérisé en ce que la rotation de la colonne est assurée par une portion de couronne sur laquelle s'enroule la chaîne d'entraînement, couronne assujettie à un chariot et liée à la colonne par un pion d'entraînement, le pion d'entraînement étant placé entre l'axe de colonne et la source de rayons X du système d'imagerie.

4. Dispositif d'examen selon les revendications 1, 2 et 3, caractérisé en ce que les déplacements de rotation et de translation de la colonne sont assurés par un frein double alterné bloquant soit la rotation de la colonne pour son déplacement en translation, soit la translation de la colonne pour son déplacement en rotation.

FIG. 1

FIG. 2

1-II-PHF 88-543

FIG. 3a

FIG. 3b

2-II-PHF 88-543

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 015 612 (PHILIPS PATENTVERWALTUNG GMBH)<br>* page 4, ligne 31 - page 10, ligne 10; figures 1-7 *<br>--- | 1 | A 61 B 6/04<br>A 61 B 6/00 |
| Y | DE-A-2 922 960 (NRT NORDISK TOENTGEN TEKNIK APS)<br>* page 1, lignes 1-27; figure 1 *<br>--- | 1 | |
| A | GB-A-2 095 080 (NATIONAL EQUIPEMENT RX)<br>* page 2, ligne 78 - page 3, ligne 91; figures 1-3 *<br>----- | 1 | |

DOMAINES TECHNIQUES
RECHERCHES (Int. Cl.4)

A 61 B 6/00

**Le présent rapport a été établi pour toutes les revendications**

| Lieu de la recherche | Date d'achèvement de la recherche | Examinateur |
|---|---|---|
| BERLIN | 25-08-1989 | WEIHS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)